# EUROPEAN PATENT SPECIFICATION

(11) **EP 1 545 455 B1**
(45) Date of publication and mention of the grant of the patent: **30.04.2008**
(21) Application number: 03769327.2
(22) Date of filing: 24.09.2003
(51) Int. Cl.: A61K 9/00, A61K 9/22, A61K 9/36

(54) **CONTROLLED DELIVERY SYSTEM FOR BIOACTIVE SUBSTANCES**
SYSTEM MIT KONTROLLIERTER FREISETZUNG BIOAKTIVER WIRKSTOFFE
SYSTÈME À LIBÉRATION CONTROLÉE DE SUBSTANCES BIOACTIVES

(30) Priority: 30.09.2002 GB 0222612
(43) Date of publication of application: 29.06.2005
(73) Proprietor: Universiteit Gent, 9000 Gent (BE)
(72) Inventor: REMON, Jean Paul, B-9090 Melle (BE); MEHUYS, Els, B-9000 Gent (BE); VERVAET, Chris, B-8870 Izegem (BE)
(74) Representative: Bird, Ariane
(86) International application number: PCT/EP2003/010715
(87) International publication number: WO 2004/028503

(56) References cited:
- WO-A-01/05376
- WO-A-89/09066
- WO-A-99/51208
- WO-A-99/63971
- US-A- 5 213 808

## Description

The present invention relates to a controlled delivery system for bioactive substances. More specifically this invention relates to composite solid shaped articles for the sustained release of biologically active ingredients, preferably composite articles comprising an outer layer and an inner core filling the outer layer. The invention additionally relates to a process for making the core material of such controlled delivery or sustained release systems, to biologically active products comprising them and to their use in agronomic and therapeutic applications.

### BACKGROUND OF THE INVENTION

Hot stage extrusion is a technique derived from the polymer and food industry. The pharmaceutical industry also took interest in this technology and during the last 10 years intensive research has been performed to explore the possibilities and drawbacks of hot stage extrusion as a new production technique for matrix formulations into which a drug is embedded. The major advantage over the more conventional matrix production methods is the continuity of the production process. Furthermore this technique is characterized by a high throughput and low material loss, a good homogeneity of the products, the absence of organic solvents in the production process and the possibility to minimize the use of excipients.

GB-A-2,249,957 discloses a controlled release composition comprising an extruded core of biologically active substance and excipients, said core being coated with a water-insoluble material, wherein:
the core is formed by extrusion from a wet mass comprising, in addition to the biologically active substance, (a) a dry powder excipient mixture comprising microcrystalline cellulose, optionally clay, further optionally a water-soluble polymeric binder (e.g. gelatin or starch) and further optionally other conventional excipients (e.g. calcium carbonate, barium sulfate, lactose or carboxymethylcellulose) and (b) water or a non-aqueous liquid;
the coating, which may extend over the entire surface of the extrudate, provided that it is sufficiently water permeable to facilitate the active substance release, may include a cellulose derivative (such as ethylcellulose) or a polymethylmethacrylate and optionally a plasticizer.
Where a portion of the extrudate is uncoated at least some of the active substance may be released by erosion of the core.

The controlled release composition of GB-A-2,249,957 does not include a lipophilic phase in the core portion thereof.

International Patent Application published as WO 95/22962 discloses a composition for the controlled delivery of an active substance into an aqueous medium by erosion at a preprogrammed rate of at least one surface of the composition, comprising:
(a) a matrix comprising the active substance, the matrix being erodible in the aqueous medium, and
(b) a coating having at least one opening exposing at least one surface of said matrix, the coating comprising (i) a thermoplastic water-insoluble first cellulose derivative and (ii) at least one of a plasticizer, a filler and a second cellulose derivative which is soluble or dispersible in water,
said coating being erodible, upon exposure to an aqueous medium, at a rate which is equal to or slower than the erosion rate of the matrix in the aqueous medium, allowing exposure of said surface of the matrix to the aqueous medium to be controlled. The first cellulose derivative of the coating may be a cellulose ether (e.g. ethylcellulose). The matrix may be a polyethylene glycol polymer with a melting point of 40-80°C or, alternatively, of the same type as the coating, i.e. comprising a thermoplastic insoluble cellulose derivative (e.g. ethylcellulose) and at least one of a plasticizer, a filler and a second cellulose derivative.

The composition of WO 95/22962 may be produced by co-extrusion of the coating with the matrix. However the controlled delivery composition of WO 95/22962 does not include a lipophilic phase in the matrix part thereof.

US-A-6,309,665 discloses a composition comprising:
(a) a system which is self-emulsifying on contact with a physiological fluid, comprising:
   - an active agent,
   - a lipophilic phase with a HLB of less than 16, consisting of a mixture of mono-, di- and triglycerides and of C₈-C₁₈ fatty acids and of polyethylene glycol monoesters and diesters,
   - a glyceride-based surfactant with a HLB of less than 16, and
   - a co-surfactant, the surfactant/co-surfactant ratio being between 0.5 and 6, and
(b) an inert polymer matrix representing from 0.5% to 40% by weight of the total composition and being able to form, in contact with a physiological fluid, a gel from which the active agent is released by diffusion. A hydrophobic polymer (e.g. ethylcellulose) is used as the polymer matrix when the active agent is hydrophilic, whereas a hydrophilic polymer (e.g. hydroxypropylmethylcellulose) is used when the active agent is hydrophobic. Example 2 of US-A-6,309,665 discloses a hydroxypropylmethylcellulose/lipophilic phase weight ratio of 0.65.

Manufacturing the composition of US-A-6,309,665 (which is in liquid or semi-solid form at room temperature) involves first preparing the self-emulsifying system, and then gradually dispersing the polymer matrix in powder form in the said self-emulsifying system. The composition of US-A-6,309,665 is in a physical state which is clearly not extrudable at room temperature and does not form part of a core/coating drug delivery system.

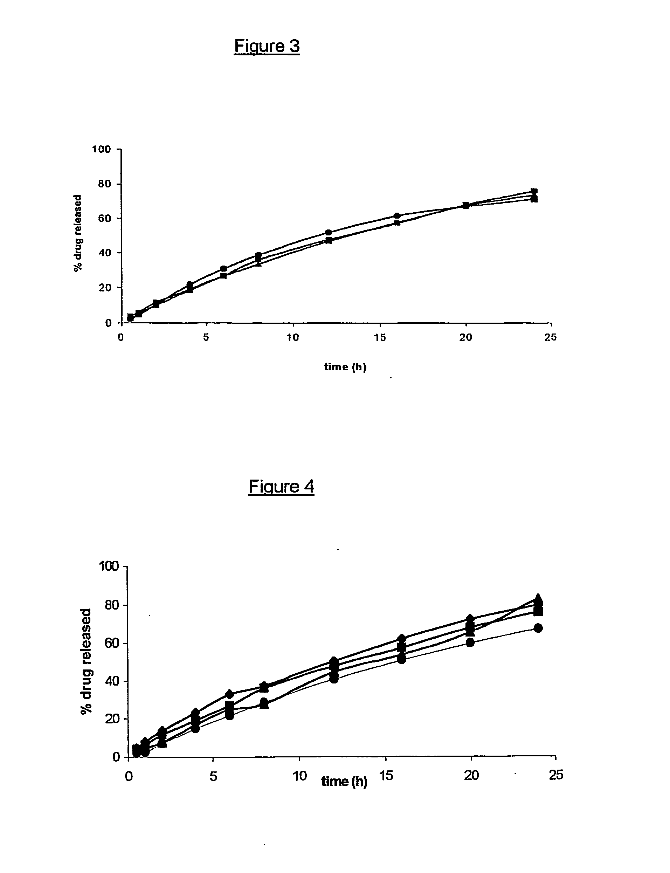

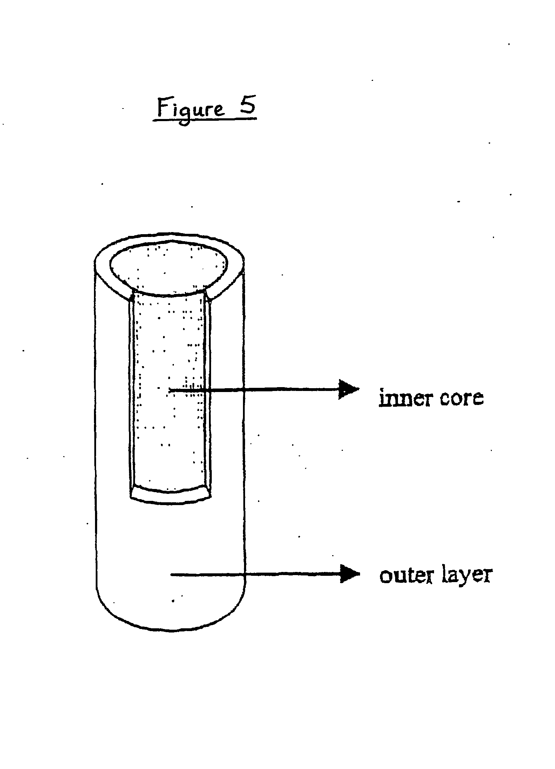

WO 02/05788 discloses a "double matrix" system comprising an outer layer, for instance in the form of a pipe or tube, and an inner core fitted into and/or filling the said outer layer, wherein the main biologically inactive components of the outer layer and of the inner core are suitably selected in order to allow diffusion of water and water-based body fluids into the core while simultaneously being able to provide controlled release of a biologically active (agronomical or pharmaceutical) ingredient included in the system. More specifically, WO 02/05788 provides a biologically active composite solid shaped article comprising:
a) an outer layer comprising:
   - at least a layer component selected from a starch component, a cellulose derivative and an acrylate (co)polymer, and
   - optionally a plasticizer for the said layer component, and
b) an inner core filling or fitted into the said outer layer and comprising:
   - at least a biologically active ingredient,
   - at least one core component selected from a starch, a lipophilic material, a cellulose derivative and an acrylate (co)polymer, and
   - optionally a plasticizer for the said core component.
Manufacturing this composite article involves extrusion or co-extrusion at a temperature from 20°C to 180°C. "Cellulose derivative " is defined in WO 02/05788 does not discriminate between hydrophobic cellulose polymers and hydrophilic cellulose polymers which like starch, when used as the main biologically inactive component of the outer layer and/or the inner core of the composite article, are able to withstand diffusion of water into the core while providing controlled release properties. Examples 1-3 and figures 3-5 of WO 02/05788 show drug release rates between about 16 and 40% after 10 hours for composite articles based on corn starch with a drug loading of 30%. Furthermore figure 5 shows that the drug release rate of these composite articles is highly dependent on the drug loading, being more than doubled when the drug loading is increased from 30% to 40%. Consequently the skilled person anticipates that the composite articles of WO 02/05788 exhibit a very low sustained release when the drug loading is above 30%. In addition, WO 02/05788 fails to disclose the use of amphiphilic materials in the inner core of the composite solid shaped article.

U.S. Patent No. 6,120,802 discloses multilayer solid drug forms comprising a co-extrudate of at least two compositions which in each case comprise a thermoplastic , pharmacologically acceptable polymeric binder being soluble or swellable in a physiological environment, and at least one of which contains a pharmaceutical active ingredient. The polymeric binder must soften or melt in the complete mixture of all components in the range from 50 to 180°C. If necessary, the polymeric binder may be plasticized by the addition of a plasticizer at a concentration up to 15% of the total weight of the composition for the particular layer, although the composition preferably comprises no plasticizer. Specific examples of US-A-6,120,802 disclose extrudates including a polyvinylpyrrolidone layer containing 30-40% by weight active ingredient and a hydroxypropylcellulose layer, both being extruded at 100-120°C. The solid drug forms of US-A-6,120,802 do not include an amphiphilic material in the core portion thereof and do not include a layer which is extrudable at low temperatures, i.e. below 50°C.

U.S. Patent No. 5,587,179 discloses a pharmaceutical formulation in the form of an effervescent or disintegrating tablet containing an active ingredient having an irritating or bitter taste, at least one matrix which delays the release of the active ingredient and which contains a fatty ester or wax, wherein the matrix is present in intimate admixture with the active ingredient and is applied to a carrier, the formulation releasing at most 65% of the active ingredient in aqueous solution at room temperature within 2 minutes but more than 70% of the active ingredient within 20 minutes at 38°C. US-A-5,587,179 thus clearly disclose what is commonly known in the art as a quick or immediate release drug formulation.

A problem addressed by the present invention is to provide sustained release systems for bio-active substances with improved characteristics, in particular pharmaceutical formulations for oral administration wherein the release of the pharmaceutically-active ingredient in the gastro-intestinal system occurs over an extended period of time of several hours. More specifically, a problem addressed by the present invention is to improve the delivery efficiency of systems including an outer layer and an inner core filling or fitted into the said outer layer while keeping the well known advantages thereof, namely their capacity to be manufactured by extrusion or co-extrusion. Another problem addressed by the present invention is to improve the delivery efficiency of systems wherein sustained drug release proceeds mainly by erosion of the core or matrix and optionally by erosion of the coating or outer layer. More specifically, problems addressed by the present invention relate to the difficult extrudability of certain cellulose derivatives such as hydroxypropylmethylcellulose, which makes them unsuitable for making pharmaceutical formulations by extrusion or co-extrusion with most drugs (especially drugs having no plasticizing properties), and the fact that most lipophilic materials described in the prior art result in a nearly immediate drug release which is not appropriate for a number of therapeutic treatments. Another problem addressed by the present invention is to provide a material or composition for making the core or matrix component of a pharmaceutical formulation, especially a composite solid shaped article, which is easily extrudable at relatively low temperatures (for instance at temperatures not above 60°C), particularly at temperatures lower than the extrusion temperatures of core or matrix materials of the prior art, while at the same time providing a sustained drug release by erosion of the said core or matrix. Yet another problem addressed by the present invention is to provide a sustained release delivery system which is widely applicable to a whole range of drugs, whatever the solubility and/or permeability of the drug as defined in the Biopharmaceutical Classification System according to G. Amidon et al. in Pharm. Res. (1995) 12:413-420.

### SUMMARY OF THE INVENTION

The present invention is based on the finding that the disadvantages of the prior art can be overcome and the various above problems can easily but surprisingly be solved by suitably designing the inner core or matrix of a biologically active composite solid shaped article, more specifically by making the said inner core from a blend of a hydrophilic cellulose polymer and an amphiphilic material in suitable proportions. At the same time the present invention provides a novel composition for making the core or matrix component of a pharmaceutical formulation, which is extrudable at low temperatures while at the same time providing a sustained drug release by erosion of the said core or matrix.

### DETAILED DESCRIPTION OF THE DRAWINGS

Figure 1 represents the drug release profiles of two open reservoir delivery systems according to embodiments of the invention, including 5% of a drug but different grades of a hydrophilic cellulose polymer in the inner core.
Figure 2 represents the drug release profiles of four open reservoir delivery systems according to embodiments of the invention including 5% by weight of a drug in the inner core but with outer layers (pipes) having different dimensions.
Figure 3 represents the drug release profiles of three open reservoir delivery systems according to embodiments of the invention, including 20% by weight of drugs with different water-solubilities in the inner core.
Figure 4 represents the drug release profiles of four open reservoir delivery systems according to embodiments of the invention with different drug loadings from 5% to 30% by weight in the inner core.
Figure 5 represents the scheme of an open reservoir delivery system according to the invention.
Figure 6 shows the plasma concentrations in a dog of a drug formulated according to this invention, compared to the same drug in a commercial retard formulation.

### DETAILED DESCRIPTION OF THE INVENTION

In a first aspect, the present invention relates to a biologically active composite solid shaped article comprising:
(a) an outer layer comprising:
   - at least one polymeric component, and
   - optionally at least one plasticizer for the said polymeric component,
(b) an inner core filling the said outer layer and comprising:
   - at least a biologically active ingredient, and
   - an excipient for the said biologically active ingredient, said excipient comprising at least one cellulose derivative,
the said composite solid shaped article being characterised in that the cellulose derivative of the inner core is a hydrophilic cellulose polymer, and the excipient of the inner core further comprises an amphiphilic material in the form of a blend with the said cellulose derivative, and the weight ratio cellulose derivative/amphiphilic material in the said blend is from about 0.2:1 to about 0.6:1.

The hydrophilic nature of the cellulose derivative is important to this invention. The term " hydrophilic " herein refers to a cellulose derivative or polymer having groups, preferably non-ionizable groups, that are capable of hydrogen bonding, in particular of association with water molecules at physiologically relevant pH. Suitable examples of hydrophilic cellulose polymers that can be used in the present invention include polymers having ether-linked substituents, for instance hydroxyalkylalkylcelluloses (wherein the alkyl group preferably has from 1 to 4 carbon atoms) such as hydroxypropylmethyl-cellulose. Hydroxypropylmethylcellulose is cellulose 2-hydroxypropyl methyl ether (hereinafter referred to as HPMC). It is a non-ionic water-soluble ether of methylcellulose which is insoluble in hot water but dissolves slowly in cold water. Being used extensively as a drug tablet excipient, HPMC is commercially available under various trade names. Suitable grades of HPMC include a low viscosity grade such as Methocel K100 from Dow Chemical, a high viscosity grade such as Methocel K100M, and other types such as the Metolose 90SH series from Shinetsu. Any other hydrophilic cellulose derivative that is barely extrudable or non-extrudable into a drug-polymer matrix, and therefore faces the problems identified herein-above, could be used as well.

The amount of the hydrophilic cellulose polymer present in the inner core of the composite solid shaped article according to the present invention depends upon the drug loading of the said core (matrix) and upon the presence of other excipients and additives but is preferably in a proportion of from about 10% to about 40%, more preferably 15% to 35%, of the total weight of the said inner core.

The nature of the amphiphilic material is important as well to this invention. The term " amphiphilic " herein refers to a material having both a hydrophobic portion, for instance comprising aliphatic or aromatic hydrocarbon groups, and a hydrophilic portion. Suitable examples of such amphiphilic materials include those having both a portion derived from a glyceride and a portion derived from a polyethylene glycol ester. For instance, it is suitable to use polyglycosylated glycerides as an amphiphilic material according to the present invention. The expression "polyglycosylated glycerides" as used herein denotes a mixture of mono-, di- and triglycerides with polyethylene glycol (PEG) mono- and diesters of C₈-C₁₈ fatty acids with a molecular weight preferably between about 200 and about 600, optionally further including glycerol and/or free PEG, the hydrophilic-lipophilic balance (HLB) value of which is controlled by the chain length of the PEG and the melting point of which is controlled by the chain length of the fatty acids, of the PEG and of the degrees of saturation of the fatty chains, and thus of the starting oil. Similarly the expression " C₈-C₁₈ fatty acids" as used herein denotes mixtures in various proportions of caprylic acid, capric acid, lauric acid, myristic acid, palmitic acid and stearic acid, when these acids are saturated, and the corresponding unsaturated acids. As is well known to the skilled person, the proportions of these fatty acids may vary as a function of the starting oils. Examples of the latter include, but are not limited to, saturated polyglycolized C₈-C₁₀ glycerides, such as the PEG-8 caprylate/caprate glyceride esters sold by Gattefosse Corporation under the tradename Labrasol; PEG-6 caprylic/capric glycerides sold by Huls Aktiengesellschaft under the trade name Softigen 767; PEG-60 corn glycerides sold by Croda under the trade name Crovol M-70; Ceteareth-20 sold by Henkel Corporation under the trade name Eumulgin B2; diethyleneglycol monoethyl-ethers sold by Gattefosse Corporation under the trade name Transcutol; a mixture of C₈-C₁₈ saturated polyglycosylated glycerides having a melting point within a range of about 42-48°C and a HLB within a range of about 8 to 16 such as sold by Gattefosse Corporation under the trade names Gelucire 48/09, Gelucire 44/14 and Gelucire 42/12; and mixtures thereof in various proportions.

The amount of the amphiphilic material present in the inner core of the composite solid shaped article according to the present invention depends upon the drug loading of the said core (matrix) and upon the presence of other excipients and additives but the said amount is preferably in a proportion of from about 30% to about 85%, more preferably 45% to 70%, of the total weight of the said inner core.

The weight ratio of the hydrophilic cellulose polymer to the amphiphilic material in the blend of the inner core of the composite solid shaped article according to the present invention is an important parameter of this invention and should be selected within a range from about 0.2:1 to about 0.6:1, preferably from 0.3:1 to 0.6:1 and more preferably from 0.3:1 to 0.5:1. The selection of the appropriate value of this parameter is within the knowledge of the skilled person and depends upon circumstances such as the required drug release profile, drug solubility, nature of the specific hydrophilic cellulose polymer and amphiphilic material being used, and so on.

The content of the biologically active agent in the inner core is not a critical parameter of this invention. It is typically in a range from about 0.1 to about 50% by weight, preferably from 0.5 to 40% by weight and more preferably from 5 to 30% by weight of the inner core, depending on the agent solubility, biologically active dose, size of the solid shaped article and similar factors well known to those skilled in the art. This content typically permits the formation of a solid solution, a term which is familiar to the skilled person. In a solid solution of a biologically active ingredient in a polymer, the active ingredient is present in the form of a molecular dispersion in the said polymer.

The term "biologically active ingredient "as used herein refers to therapeutic, diagnostic, cosmetic and prophylactic agents as well as other agents, e.g. selected from insecticides, pesticides, herbicides, plant growth regulators, fertilisers, crop treatment agents, anti-microbial agents (in particular fungicides and bactericides), admissible for use in plants, animals and humans. Thus the biologically active article of this invention may be for pharmaceutical use, cosmetic use, veterinary use or for plant treatment. The therapeutic agent can be selected for its specific properties such as for instance its anti-thrombotic, anti-inflammatory, anti-proliferative or anti-microbial efficiency. The latter include for instance anti-microbial agents such as broad spectrum antibiotics for combating clinical and sub-clinical infection, for example gentamycin, vancomycine and the like. Other suitable therapeutic agents are naturally occurring or synthetic organic or inorganic compounds well known in the art, including non-steroidal anti-inflammatory drugs, proteins and peptides (produced either by isolation from natural sources or recombinantly), hormones (for example androgenic, estrogenic and progestational hormones such as oestradiol), bone repair promoters, carbohydrates, antineoplastic agents, antiangiogenic agents, vasoactive agents, anticoagulants, immunomodulators, cytotoxic agents, antiviral agents, antibodies, neurotransmitters, oligonucleotides, lipids, plasmids, DNA and the like. Suitable therapeutically active proteins include e.g. fibroblast growth factors, epidermal growth factors, platelet-derived growth factors, macrophage-derived growth factors such as granulocyte macrophage colony stimulating factors, ciliary neurotrophic factors, tissue plasminogen activator, B cell stimulating factors, cartilage induction factor, differentiating factors, growth hormone releasing factors, human growth hormone, hepatocyte growth factors, immunoglobulins, insulin-like growth factors, interleukins, cytokines, interferons, tumor necrosis factors, nerve growth factors, endothelial growth factors, osteogenic factor extract, T cell growth factors, tumor growth inhibitors, enzymes and the like, as well as fragments thereof. Suitable diagnostic agents include conventional imaging agents (for instance as used in tomography, fluoroscopy, magnetic resonance imaging and the like) such as transition metal chelates. Suitable anti-microbial agents include e.g. halogenated phenols, chlorinated diphenylethers, aldehydes, alcohols such as phenoxyethanol, carboxylic acids and their derivatives, organometallic compounds such as tributyltin compounds, iodine compounds, mono- and polyamines, sulfonium and phosphonium compounds; mercapto compounds as well as their alkaline, alkaline-earth and heavy metal salts; ureas such as trihalocarbanilide, isothia- and benzisothiazolone derivatives. Suitable insecticides include natural ones, e.g. nicotine, rotenone, pyrethrum and the like, and synthetic ones like chlorinated hydrocarbons, organophosphorus compounds, biological insecticides (e.g. products derived from *Bacillus thuringiensis*), synthetic pyrethroids, organosilicon compounds, nitro-imines and nitromethylenes. Suitable fungicides include e.g. dithiocarbamates, nitrophenol derivatives, heterocyclic compounds (including thiophtalimides, imidazoles, triazines, thiadiazoles, triazoles and the like), acylalanines, phenylbenzamides and tin compounds. Suitable herbicides include e.g. trichloroacetic and aromatic carboxylic acids and their salts, substituted ureas and triazines, diphenyl ether derivatives, anilides, uraciles, nitriles and the like. Suitable fertilizers include e.g. ammonium sulphate, ammonium nitrate, ammonium phosphate and the like, and mixtures thereof.

Therapeutically active agents which are advantageously incorporated into the composite solid shaped articles of the present invention belong to all permeability and solubility classes of the Biopharmaceutical Classification System according to G. Amidon et al. (cited *supra).* As will be appreciated by those skilled in the art, these drugs belong to various therapeutic classes including, but are not limited to, β-blockers, calcium antagonists, ACE inhibitors, sympathomimetic agents, hypoglycaemic agents, contraceptives, α-blockers, diuretics, anti-hypertensive agents, antipsoriatics, bronchodilators, cortisones, anti-mycotics, salicylates, cytostatics, antibiotics, virustatics, antihistamines, UV-absorbers, chemotherapeutics, antiseptics, estrogens, scar treatment agents, antifungals, antibacterials, antifolate agents, cardiovascular agents, nutritional agents, antispasmodics, analgesics and the like.

The invention is suitable, for example, for formulating the following therapeutically active ingredients or cosmetic agents: acebutolol, acetylcysteine, acetylsalicylic acid, acyclovir, alfuzosine, alprazolam, alfacalcidol, allantoin, allopurinol, alverine, ambroxol, amikacin, amiloride, aminoacetic acid, amiodarone, amitriptyline, amlodipine, amoxicillin, ampicillin, ascorbic acid, aspartame, astemizole, atenolol, beclomethasone, benserazide, benzalkonium hydrochloride, benzocaine, benzoic acid, betamethasone, bezafibrate, biotin, biperiden, bisoprolol, bromazepam, bromhexine, bromocriptine, budesonide, bufexamac, buflomedil, buspirone, caffeine, camphor, captopril, carbamazepine, carbidopa, carboplatin, cefachlor, cefalexin, cefatroxil, cefazolin, cefixime, cefotaxime, ceftazidime, ceftriaxone, cefuroxime, cephalosporins, cetirizine, chloramphenicol, chlordiazepoxide, chlorhexidine, chlorpheniramine, chlortalidone, choline, cyclosporin, cilastatin, cimetidine, ciprofloxacin, cisapride, cisplatin, clarithromycin, clavulanic acid, clomipramine, clonazepam, clonidine, clotrimazole, codeine, cholestyramine, cromoglycic acid, cyanocobalamin, cyproterone, desogestrel, dexamethasone, dexpanthenol, dextromethorphan, dextropropoxiphen, diazepam, diclofenac, digoxin, dihydrocodeine, dihydroergotamine, dihydroergotoxin, diltiazem, diphenhydramine, dipyridamole, dipyrone, disopyramide, domperidone, dopamine, doxycycline, enalapril, ephedrine, epinephrine, ergocalciferol, ergotamine, erythromycin, estradiol, ethinylestradiol, etoposide, Eucalyptus globulus, famotidine, felodipine, fenofibrate, fenoterol, fentanyl, flavine mononucleotide, fluconazole, flunarizine, fluorouracil, fluoxetine, flurbiprofen, furosemide, gallopamil, gemfibrozil, Ginkgo biloba, glibenclamide, glipizide, clozapine, Glycyrrhiza glabra, griseofulvin, guaifenesin, haloperidol, heparin, hyaluronic acid, hydrochlorothiazide, hydrocodone, hydrocortisone, hydromorphone, ipratropium hydroxide, ibuprofen, imipenem, indomethacin, iohexol, iopamidol, isosorbide dinitrate, isosorbide mononitrate, isotretinoin, ketotifen, ketoconazole, ketoprofen, ketorolac, labetalol, lactulose, lecithin, levocarnitine, levodopa, levoglutamide, levonorgestrel, levothyroxine, lidocaine, lipase, imipramine, lisinopril, loperamide, lorazepam, lovastatin, medroxyprogesterone, menthol, methotrexate, methyldopa, methylprednisolone, metoclopramide, metoprolol, miconazole, midazolam, minocycline, minoxidil, misoprostol, morphine, N-methylephedrine, naftidrofuryl, naproxen, neomycin, nicardipine, nicergoline, nicotinamide, nicotine, nicotinic acid, nifedipine, nimodipine, nitrazepam, nitrendipine, nizatidine, norethisterone, norfloxacin, norgestrel, nortriptyline, nystatin, ofloxacin, omeprazole, ondansetron, pancreatin, panthenol, pantothenic acid, paracetamol, paroxetine, penicillins, phenobarbital, pentoxifylline, phenoxymethylpenicillin, phenylephrine, phenylpropanolamine, phenytoin, physostigmine, piroxicam, polymyxin B, povidone iodine, pravastatin, prazepam, prazosin, prednisolone, prednisone, bromocriptine, propafenone, propranolol, proxyphylline, pseudoephedrine, pyridoxine, quinidine, ramipril, ranitidine, reserpine, retinol, riboflavin, rifampicin, rutoside, saccharin, salbutamol, salcatonin, salicylic acid, simvastatin, somatotropin, sotalol, spironolactone, sucralfate, sulbactam, sulfamethoxazole, sulfasalazine, sulpiride, tamoxifen, tegafur, teprenone, terazosin, terbutaline, terfenadine, tetracaine, tetracycline, theophylline, thiamine, ticlopidine, timolol, tranexamic acid, tretinoin, triamcinolone acetonide, triamterene, triazolam, trimethoprim, troxerutin, uracil, valproic acid, verapamil, folinic acid, zidovudine, zopiclone, and enantiomers thereof, organic and inorganic salts thereof, hydrates thereof and mixtures thereof, in particular mixtures in synergistic proportions.

Other active ingredients for the purpose of the invention are vitamins, include those of the A group, of the B group (which means, besides B1, B2, B6 and B12, also compounds with vitamin B properties such as adenine, choline, pantothenic acid, biotin, adenylic acid, folic acid, orotic acid, pangamic acid, carnitine, p-aminobenzoic acid, myo-inositol and lipoic acid), vitamin C, vitamins of the D group, E group, F group, H group, I and J groups, K group and P group.

According to this invention, the biologically active ingredient or drug may be classifiable as Class II (poorly soluble, highly permeable) or Class IV (poorly soluble, poorly permeable) of the Biopharmaceutical Classification System according to G. Amidon et al. (cited *supra)* and may have a water-solubility below about 2.5 mg/ml, even between 0.1 and 1 mg/ml (i.e. " very slightly soluble " as defined in the United States Pharmacopeia), even below 0.1 mg/ml (i.e. " practically insoluble "as defined in the United States Pharmacopeia), even below about 5 µg/ml and may even have a water-solubility as low as about 0.2 µg/ml, at room temperature and physiological pH. Non-limiting examples of such therapeutically active agents or drugs include for instance, but are not limited to, chlorothiazide, hydrochlorothiazide, nimodipine, flufenamic acid, furosemide, mefenamic acid, bendroflumethiazide, benzthiazide, ethacrinic acid, nitrendipine, itraconazole, saperconazole, troglitazone, prazosin, atovaquone, danazol, glibenclamide, griseofulvin, ketoconazole, carbamazepine, sulfadiazine, florfenicol, acetohexamide, ajamaline, benzbromarone, benzyl benzoate, betamethasone, chloramphenicol, chlorpropamide, chlorthalidone, clofibrate, diazepam, dicumarol, digitoxin, ethotoin, glutethimide, hydrocortisone, hydroflumethiazide, hydroquinine, indomethacin, ibuprofen, ketoprofen, naproxen, khellin, nitrazepam, nitrofurantoin, novalgin, oxazepam, papaverine, phenylbutazone, phenytoin, prednisolone, prednisone, reserpine, spironolactone, sulfabenzamide, sulfadimethoxine, sulfamerazine, sulfamethazine, sulfamethoxypyridazine, succinylsulfathiazole, sulfamethizole, sulfamethoxazole (also in admixture with trimethoprim), sulfaphenazole, sulfathiazole, sulfisoxazole, sulpiride, testosterone and diaminopyrimidines. Suitable examples of such diaminopyrimidines include, but are not limited to, 2,4-diamino-5-(3,4,5-trimethoxybenzyl) pyrimidine (known as trimethoprim), 2,4-diamino-5-(3,4-dimethoxybenzyl) pyrimidine (known as diaveridine), 2,4 diamino-5-(3,4,6-trimethoxybenzyl) pyrmidine, 2,4-diamino-5-(2-methyl-4,5-dimethoxybenzyl) pyrimidine (known as ormetoprim), 2,4-diamino-5-(3,4-dimethoxy-5-bromobenzyl) pyrimidine, and 2,4-diamino-5-(4-chloro-phenyl)-6-ethylpyrimidine (known as pyrimethamine). As will be appreciated by those skilled in the art, these drugs belong to various therapeutic classes, including diuretics, anti-hypertensive agents, anti-viral agents, antibacterial agents, antifungals, etc, and are not limited to human or veterinary use alone.

The amount (dose) of the biologically active agent in the solid shaped article of this invention preferably is an amount sufficient for providing the desired biologic activity in plants, animals or humans, e.g. in the prevention or treatment of a disorder or disease for which the therapeutic agent is used. A suitable amount is typically in a range from about 0.1 to 200 mg, preferably from 5 to 100 mg, more preferably from 20 to 80 mg, depending upon parameters such as, but not limited to, the nature of the biologic agent involved and (for therapeutic agents) the age of the patient (typically lower doses are used for pediatrics) to which the said agent will be administered.

The constitution of the outer layer (coating) of the biologically active composite solid shaped article is not critical to the present invention. Such coatings are well known in pharmaceutical technology and thus any extrudable coating that will retain the structural integrity of the inner core may be used. The outer layer (coating) usually comprises at least one polymeric component and optionally at least one plasticizer for the said polymeric component. Suitable polymeric components for the coating include hydrophobic cellulose polymers, such as cellulose ethers. A preferred cellulose ether is ethylcellulose, typically an ethylcellulose with an ethoxyl content in the range of about 44 to about 53%. Typical commercially available ethylcellulose products have such ethoxyl contents, corresponding to about 2.2 to 2.7 ethoxyl groups per anhydroglucose unit. Other suitable coating polymeric components which are shapeable by extrusion upon heating include for instance methylcellulose, acrylate (co)polymers, polyvinylpyrrolidone, polyethylene oxide, polyvinyl alcohol, poly(ethylene-co-vinyl acetate) and the like, and mixtures thereof. Preferably the polymeric component for the outer layer (coating) should be a pharmaceutically acceptable grade.

The term " acrylate (co)polymer " as used herein refers to homopolymers and copolymers of at least one C₁₋₁₀alkyl or C₁₋₁₀alkylamino acrylate or methacrylate, further optionally containing a minor amount (up to about 10%) of a hydrophilic acrylic monomer such as acrylic or methacrylic acid. Non-limiting examples thereof are polyethylacrylate, polymethylmethacrylate and the like.

Additionally plasticizers may be included in the outer layer (coating) of the biologically active composite solid shaped article of the invention. The term " plasticizer "as used herein refers to compounds such as glycerol, polyols (namely tetraols, pentols and hexols such as sorbitol), esters formed between glycerol and acetic acid (e.g. triacetine), sugars, glycol glycoside, poly(ethylene glycol), fatty acids and esters thereof with polyethylene glycol, propylene glycol, butylene glycol, phtalate esters, sebacate esters and the like, and mixtures thereof. The nature and amount of the specific plasticizer to be used will vary, in a manner well known to those skilled in the art, depending on the specific outer layer (coating) polymeric component to be plasticized. The plasticizer amount is typically in a range from 0 to 40% by weight, preferably from 5 to 30% by weight, more preferably from 10 to 25% by weight, with respect to the weight of the polymeric component.

Each of the inner core (matrix) and of the outer layer (coating) may further comprise one or more further (preferably pharmaceutically acceptable) excipients such as emulsifiers or surface-active agents, thickening agents, gelling agents or other additives. The said excipients may be independently selected for the core and the outer layer, in order to impart specific characteristics to one of them, or both, as is well known in the art.

Suitable emulsifiers or surface-active agents include water-soluble natural soaps and water-soluble synthetic surface-active agents. Suitable soaps include alkaline or alkaline-earth metal salts, unsubstituted or substituted ammonium salts of higher fatty acids (C₁₀-C₂₂), e.g. the sodium or potassium salts of oleic or stearic acid, or of natural fatty acid mixtures obtainable form coconut oil or tallow oil. Synthetic surface-active agents (surfactants) include anionic, cationic and non-ionic surfactants, e.g. sodium or calcium salts of polyacrylic acid; sulphonated benzimidazole derivatives preferably containing 8 to 22 carbon atoms; alkylarylsulphonates; and fatty sulphonates or sulphates, usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide) and the like.

Suitable emulsifiers further include partial esters of fatty acids (e.g. lauric, palmitic, stearic or oleic) or hexitol anhydrides (e.g., hexitans and hexides) derived from sorbitol, such as commercially available polysorbates. Other emulsifiers which may be employed include, but are not limited to, materials derived from adding polyoxyethylene chains to non-esterified hydroxyl groups of the above partial esters, such as Tween 60 commercially available from ICI Americas Inc.; and the poly(oxyethylene) poly(oxypropylene) materials marketed by BASF under the trade name Pluronic.

Structure-forming, thickening or gel-forming agents may be included into the inner core and/or the outer layer of the composite solid shaped article of the invention. Suitable such agents are in particular highly dispersed silicic acid, such as the product commercially available under the trade name Aerosil; bentonites; tetraalkyl ammonium salts of montmorillonites (e.g., products commercially available under the trade name Bentone), wherein each of the alkyl groups may contain from 1 to 20 carbon atoms; cetostearyl alcohol and modified castor oil products (e.g. the product commercially available under the trade name Antisettle).

Gelling agents which may be included in the inner core and/or the outer layer of the composite solid shaped article of the present invention include, but are not limited to, cellulose derivatives such as carboxymethylcellulose, cellulose acetate and the like; natural gums such as arabic gum, xanthum gum, tragacanth gum, guar gum and the like; gelatin; silicon dioxide; synthetic polymers such as carbomers, and mixtures thereof. Gelatin and modified celluloses represent a preferred class of gelling agents.

Other optional excipients which may be included in the inner core and/or the outer layer of the composite solid shaped article include additives such as magnesium oxide; azo dyes; organic and inorganic pigments such as titanium dioxide; UV-absorbers; stabilisers; odor masking agents; viscosity enhancers; antioxidants such as, for example, ascorbyl palmitate, sodium bisulfite, sodium metabisulfite and the like, and mixtures thereof; preservatives such as, for example, potassium sorbate, sodium benzoate, sorbic acid, propyl gallate, benzylalcohol, methyl paraben, propyl paraben and the like; sequestering agents such as ethylene-diamine tetraacetic acid; flavoring agents such as natural vanillin; buffers such as citric acid and acetic acid; extenders or bulking agents such as silicates, diatomaceous earth, magnesium oxide or aluminum oxide; densification agents such as magnesium salts; and mixtures thereof.

The selection of the optimal excipients and their proportion in the inner core and/or the outer layer of the composite solid shaped article of the present invention depends on the specific biologically-active ingredient to be formulated and of the required drug release characteristics and is well known to the skilled person

The biologically active composite solid shaped article of this invention may have any shape such as cylindrical, ellipsoidal, tubular, sheet-like (for example for transdermal therapeutic applications) or similar, i.e. its section may be circular, elliptic, square, rectangular or the like. It may have any dimension usually suitable for the delivery of a biologically active ingredient for a specific agronomic or therapeutic application. For instance, when it is intended to be used for a pharmaceutical or veterinary application for administration to a human or an animal (e.g. a mammal), the inner core should preferably have a dimension from about 0.1 to 10 cm, more preferably from 0.1 to 1 cm, and/or the outer layer should preferably have a thickness from 0.1 to 5 cm, more preferably from 0.1 to 1 cm. The relative dimension of the outer layer (i.e. its thickness), with respect to the dimension of the inner core, is not critical to the present invention and may be higher or lower than or equal to the same.

The outer layer and the inner core (such as described in detail in the first aspect of the invention) of the composite solid shaped article may both be produced by means of extrusion. They can either be made separately and then assembled manually or automatically, or the inner core can be spouted into the outer layer manually or automatically, or preferably they can be made and assembled simultaneously into a composite solid shaped article by means of co-extrusion. Summarizing the process for making the composite solid shaped article of this invention, the outer layer (coating) may be formed by extrusion of its components at a temperature within a range from about 20°C to about 180°C, preferably from 70°C to 140°C, depending on the nature of its polymeric component and of the optional plasticizer and other excipients. The inner core (matrix) of the composite article may be formed, and this is an unexpected advantage over the prior art, by extruding a mixture or blend of its components at a relatively low temperature within a range from about 20°C to about 60°C, preferably from 20°C to 45°C, depending on the specific hydrophilic cellulose polymer, the specific amphiphilic material and the specific drug loading being selected. Thus in another aspect the present invention relates to a process for making the core material of a biologically active article as defined hereinabove in a first aspect of the present invention, comprising extruding a blend of at least a biologically active ingredient, at least a hydrophilic cellulose polymer and at least an amphiphilic material (these terms being defined as herein-above), the weight ratio of the hydrophilic cellulose polymer to the amphiphilic material in the said blend being from about 0.2:1 to about 0.6:1, at a temperature within the range from about 20°C to about 60°C.

Before co-extrusion of the inner core (matrix) and the outer layer (coating), a composition must be prepared separately for each portion of the biologically active composite solid shaped article. For this purpose, the starting components of each composition may be processed in a separate extruder or melt container with downstream gear pump. This entails the components being fed in singly or as dry mixture continuously (e,g. via differential weigh feeders). Then mixing and/or softening or melting of the composition takes place in the said extruder or melt container. If it is desired to incorporate a particular temperature-sensitive active ingredient, this is expediently added only after the softening or melting of the composition and is incorporated by longitudinal and transverse mixing in the extruder or in a kneader or mixing reactor and homogenized with the composition. An extruder, especially a twin screw extruder or a single screw extruder with a mixing section, is particularly expedient for preparing such a composition because this permits operation under conditions which are optimal for the specific starting material involved. For example, as exemplified above, different processing temperatures can be selected for each portion of the composite article. The molten or plastic compositions from the individual extruders or other units may then be passed into a joint co-extrusion die, and extruded. The shape of the co-extrusion die depends on the form or geometry required for the composite solid shaped article. For example, dies with a plain die gap (so-called slot dies) and dies with an annular slit are suitable for this purpose. The die design may moreover depend upon the polymeric component used in the composition.

Shaping to the required composite article takes place downstream of the co-extrusion die. It is possible to produce a large number of shapes, depending on the co-extrusion die and the type of shaping. For example, open multilayer tablets can be produced from an extrudate from a slot die, which has two or more layers, by punching or cutting out, e.g. using an incandescent wire. Alternatively, open reservoir systems or matrix-in-cylinder systems such as multilayer tablets can be produced via a die with an annular slit by cutting or chopping the extrudate immediately after extrusion or, preferably, after at least partial cooling thereof.

Closed solid shaped articles, e.g. drug forms in which the core containing the biologically active ingredient is completely surrounded by an outer layer, may be obtained in particular using a die with an annular slit by treating the extrudate in a suitable pinch device.

Each of the inner core (matrix) or outer layer (coating) compositions can be extruded through circular, elliptical or annular dies. Thus, the extruded outer layer may be circular or annular in cross-section, e.g. in the form of hollow tubes or pipes. Typically such tubes will have an external diameter of from 1 mm to about 20 mm, preferably from 2 mm to 10 mm. The form of the open reservoir systems (as shown in figure 5) can be varied for instance as follows:
- Cylindrical:: cylindrical die: 0.1 cm to 5 cm
tubular die: 0.1 cm to 5 cm (wall diameter)
- Ellipsoidal:: ellipsoidal die: 0.5 cm to 10 cm (width) - 0.1 cm to 5 cm
(height)
ellipsoidal pipe die: 0.1 cm to 5 cm (wall diameter)

The extruded lengths of material may be cut into appropriate lengths to produce the appropriate dosage forms. The geometrical dimensions will, of course, be dependent on the intended use. Thus, for use in humans they will be of a size suitable for swallowing, whereas for animal use they may be correspondingly larger. Suitable lengths for human use are from about 5 to about 20 mm.

The extrusion process may be carried out using equipment and techniques which are known in the art of extrusion processing. Examples of extrusion equipment which may be used include, but are not limited to, end plate extruders, screen extruders, rotary cylinder extruders, rotary gear extruders and ram extruders, screw extruders, disk extruders, drum extruders.

Operating parameters of the extruder, such as rate, speed and pressure, will be adjusted so as to optimize the properties of the extrudate in accordance with techniques which are familiar to those skilled in the art of extrusion. By appropriate adjustment to the force applied to cause extrusion, it is possible to obtain an extrudate under steady-state flow conditions which has an acceptably smooth surface.

In yet another aspect, the present invention relates to the use of a hydrophilic cellulose polymer (such as above-disclosed in details) in combination with an amphiphilic material (such as above-disclosed in details), wherein the weight ratio of the hydrophilic cellulose polymer to the amphiphilic material in the said combination is from about 0.2:1 to about 0.6:1, for manufacturing at least a portion of a biologically active article as defined hereinabove in a first aspect of the present invention. Preferably the said use is for making a biologically active composite solid shaped article comprising two or more portions or layers. More preferably, the said biologically active composite solid shaped article comprises an inner core made from the said combination of the invention, and further comprises one or more coating or portion (preferably an inner core or matrix) of the biologically active formulation which includes a hydrophilic cellulose polymer in combination with an amphiphilic material additionally includes from about 0.5 to 30% by weight of a biologically active ingredient.

The present invention, through the novel combination of a hydrophilic cellulose polymer with an amphiphilic material in specific proportions, provides significant and unexpected advantages in the field of biologically active ingredient formulation, such as but not limited to:
- a sustained release core (matrix) wherein the gel forming capacities of a hydrophilic cellulose polymer are able to sustain the release of a biologically active ingredient from an amphiphilic material matrix, i.e. the release occurs over an extended period of time of several hours; in particular, the biologically active composite solid shaped articles of the invention typically achieve an active ingredient release within a range of about 20% to 50% after 10 hours;
- the addition of a semi-solid, waxy amphiphilic material to a hydrophilic cellulose polymer in suitable proportions results in a mixture which can form a drug matrix by extrusion at room temperature, thus allowing the incorporation of thermosensitive drugs.

Therefore the biologically active formulations and composite solid shaped articles of this invention are well suited for oral administration in a wide range of therapeutic treatments.

Dissolution profiles of a few biologically active composite solid shaped articles of the invention are shown in the following examples which are provided for illustrative purpose only, and should in no way be interpreted as limiting the scope of the present invention.

### EXAMPLE 1

In order to obtain a system suitable for drug sustained release, we produced an "open reservoir" system consisting of a hot stage extruded ethylcellulose pipe surrounding a drug-containing Gelucire^{®}-HPMC matrix. An exemplary production procedure of all systems tested herein-after is as follows. Extrusion was performed on a MP19 TC25 laboratory intermeshing co-rotating twin screw extruder of APV-Baker (Newcastle-under-Lyme, United Kingdom). For the production of the outer layers (pipes), ethylcellulose and 20% dibutyl sebacate (based on the ethylcellulose polymer weight) acting as a plasticizer were extruded under the following conditions: screw speed of 5 rpm, powder feed rate of 0.29 kg/h and a temperature profile of 125-125-115-105-80°C from powder feed to die. The extrudates (having an internal diameter of 5 mm and a wall thickness of 1 mm) were cut into pieces of 1 to 2 cm. The inner core (matrix) mixture consisted of 5% by weight theophylline monohydrate (available from Ludeco, Belgium), 30% by weight pharmaceutical grade of HPMC (Methocel^{®} K100 or K100M available from Colorcon, United Kingdom) and 65% by weight Gelucire^{®} 44/14 (a mixture of C₈-C₁₈ saturated polyglycosylated glycerides having a melting point of 44°C and a HLB of 14 available from Gattefosse, France). Gelucire^{®} 44/14 was heated to 65°C. Theophylline monohydrate and HPMC were mixed in a mortar. The molten Gelucire^{®} was then admixed with the theophylline-HPMC mixture and homogenised. This mixture was spouted into the hollow pipes and, after cooling, excess material was cut off. Alternatively the open reservoir systems were produced by means of co-extrusion as follows. The semi-solid inner core (matrix) was extruded at 28°C, a screw speed of 25 rpm, a powder feed rate (theophylline + HPMC) of 0.8 kg/h and a molten Gelucire^{®} 44/14 addition rate of 1.5 kg/h (with a peristaltic pump).

Dissolution tests were performed while using a dissolution system consisting of a VK 7000 dissolution bath and a VK 8010 automatic sampling station (available from VanKel, United States). The paddle method (USP24) at 150 rpm and 37 ± 0.5°C was selected, using a test medium with an ionic strength of 0.14 in order to provide physiologically relevant conditions. Samples were taken at 0.5, 1, 2, 4, 6, 8, 12, 16, 20 and 24 hours and analysed spectrophotometrically.

### EXAMPLE 2

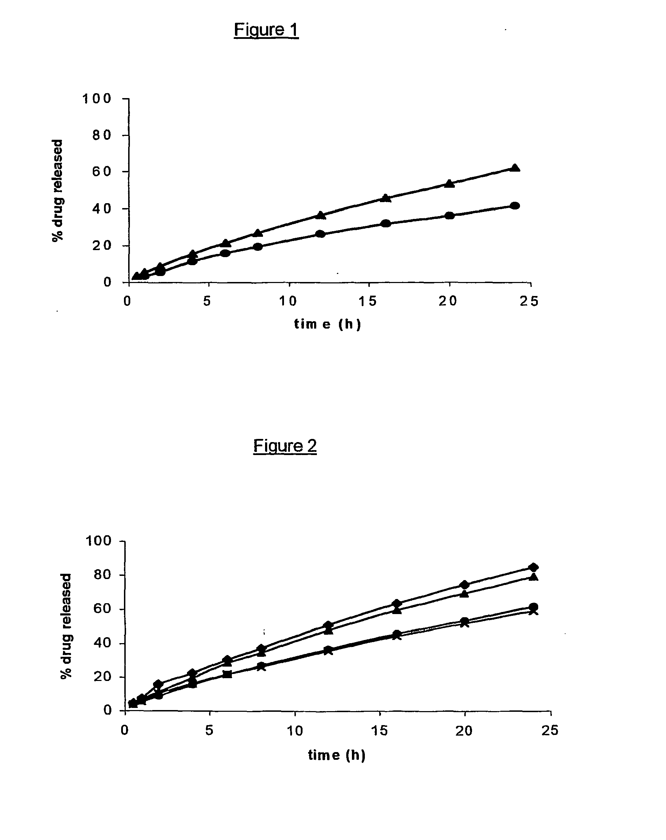

Figure 1 represents the drug release profiles of two open reservoir delivery systems (composite articles) according to the invention, each including 5% by weight theophylline monohydrate as a drug but including different grades of HPMC in the inner core. The latter consisted of 5% by weight theophylline monohydrate, 30% by weight Methocel^{®} K100 ( ▲ ) or Methocel^{®} K100M ( ● ) and 65% by weight Gelucire^{®} 44/14, and was surrounded by an outer layer (pipe) comprising a 100:20 (by weight) mixture of ethylcellulose and dibutyl sebacate. Figure 1 shows that Methocel^{®} K100M provides a more prolonged sustained release than Methocel^{®} K100, all other parameters being kept equal.

### EXAMPLE 3

Figure 2 represents the drug release profiles of four open reservoir delivery systems (composite articles) according to the invention, each including 5% by weight of the same drug in the inner core but with outer layers (pipes) having different dimensions. Each inner core (matrix) contained 5% by weight theophylline monohydrate, 30% by weight Methocel^{®} K100 and 65% by weight Gelucire^{®} 44/14, and was surrounded by an outer layer (pipe) comprising a 100:20 (by weight) mixture of ethylcellulose and dibutyl sebacate. The said pipe had, respectively, a length of 18 mm and an internal diameter of 5 mm ( • ); a length of 18 mm and an internal diameter of 7 mm ( X ); a length of 12 mm and an internal diameter of 5 mm ( ▲ ); and finally a length of 12 mm and an internal diameter of 7 mm (◆).

### EXAMPLE 4

Figure 3 represents the drug release profiles of three open reservoir delivery systems (composite articles) according to the invention, each including 20% by weight of drugs with different water-solubility in the inner core. Each inner core (matrix) consisted of 25.2% by weight Methocel^{®} K100, 54.8% by weight Gelucire^{®} 44/14 and 20% by weight of, respectively, hydrochlorothiazide ( • ), theophylline monohydrate ( ▲ ) or propranolol hydrochloride ( ■ ), and was surrounded by an outer layer (pipe) comprising a 100:20 (by weight) mixture of ethylcellulose and dibutyl sebacate. Figure 3 shows that the drug release profile from the open reservoir system (composite article) is nearly independent from the drug water-solubility.

### EXAMPLE 5

Figure 4 represents the drug release profiles of four open reservoir delivery systems (composite articles) according to the invention, each with different loadings (contents), ranging from 5% to 30% by weight, of the same drug (propranolol hydrochloride) in the inner core. The latter consisted of Methocel® K100 and Gelucire^{®} 44/14 (in a 0.46:1 ratio) and, respectively, 5% by weight drug ( • ), 10% by weight drug ( ▲ ), 20% by weight drug ( ■ ) and 30% by weight drug ( ◆ ), and was surrounded by an outer layer (pipe) comprising a 100:20 (by weight) mixture of ethyl cellulose and dibutyl sebacate. Figure 4 shows a limited effect of the drug loading (content) in the inner core on the drug release rate, contrary to the teaching of the prior art.

### EXAMPLE 6 - comparative in vivo evaluation

An *in vivo* study in dogs was performed in order to check the bioavailability and behaviour of the matrix-in-cylinder (i.e. inner core and outer layer) system of the invention and to compare it to that of a commercially available multiparticulate sustained release system with the same drug. In order to evaluate the influence of the outer layer polymeric component of the system of the invention on drug release, a hard gelatine capsule was filled with the same inner core formulation as the matrix-in-cylinder system and was also tested *in vivo.*

Six dogs (male mixed-breed, weight 35 - 41 kg) were used in this study. The three following formulations, each containing 80 mg propranolol hydrochloride as the biologically active ingredient (drug), were used:
- F-1:: a system according to the invention, wherein the inner core (matrix) consists (by weight) of 27% of the drug, 23% of Methocel^{®} K100 and 50% Gelucire^{®} 44/14, and wherein said inner core is surrounded by an ethylcellulose outer pipe (length = 12 mm, internal diameter = 5 mm).
- F-2:: a hard gelatine capsule filled with of a mixture consisting of (by weight) 27 % drug, 23 % Methocel^{®} K100 and 50 % Gelucire^{®} 44/14.
- F-3:: lnderal^{®} retard mitis, a sustained release formulation commercially available from Astra-Zeneca, Brussels, Belgium.

All three formulations were administered in a randomized cross-over sequence with a washout period of at least 7 days. On the experimental days the dogs were fasted since the previous evening, water being available ad libitum. Each formulation was orally administered together with 200 ml of water. Blood samples were obtained:
- at 0.5, 1, 2, 3, 4, 5, 6, 8, 12 and 24 hours respectively after intake of F-1 or F-3, and
- at 0.25, 0.5, 1, 1.5, 2, 3, 4, 5, 6, 8 and 12 hours respectively after intake of F-2.
The blood samples were collected in dry heparinized tubes and centrifuged for 5 minutes at 3000 rpm within 1 hour after collection. The plasma was stored at -20°C until performing the following propranolol assay procedure. No food was administered to the dogs during the blood sampling period, however water was freely available.

Plasma propranolol concentrations were determined by a high performance liquid chromatography (hereinafter referred as HPLC)-fluorescence method. All chemicals were of analytical or HPLC grade. 25 µl of an internal standard solution (1 µg/ml of 4-methylpropranolol in methanol) was evaporated to dryness under a nitrogen stream, and the residue was re-dissolved in 1 ml plasma. The drug was extracted using a solid phase extraction method (hereinafter referred as SPE). The SPE columns were conditioned consecutively with 1 ml methanol, 1ml water and 1 ml phosphate buffer saline. Next, the plasma samples were transferred quantitatively onto the SPE columns. A rinsing step was performed with 1 ml of a 60/40 (volume ratio) mixture of methanol and water; elution was performed with 1 ml methanol. The obtained eluates were evaporated to dryness under a nitrogen stream, the residue was re-dissolved in 200 µl eluent and 100 µl of the solution was injected into the chromatograph. Plasma concentrations were determined via a calibration curve, the standards for the calibration curve being treated as the samples. The HPLC equipment consisted of:
- a solvent pump (L-6000, commercially available from Hitachi, Tokyo, Japan) set at a constant flow rate of 1 ml/minute,
- a variable wavelength detector (L-7480 fluorescence detector, also commercially available from Hitachi, Tokyo, Japan) set at 250 nm (for propranolol and 4-methylpropranolol) as excitation wavelength and at 360 nm (for propranolol) or 365 nm (for 4-methyl-propranolol) as emission wavelength,
- a reversed phase column and pre-column (LiChro-CART^{®} 125-4 and 4-4, LiChrospher^{®} 60 RP-select B 5 µm, commercially available from Merck, Darmstadt, Germany), and
- an automatic integrating system (L-7000, commercially available from Hitachi, Tokyo, Japan).
The SPE equipment consisted of OASIS HLB cartridges (1 cc 30 mg, available from Waters, Brussels, Belgium) and a 16-port vacuum manifold (available from Alltech Europe, Laarne, Belgium). The eluent had the following composition: 700 ml of a buffer solution (consisting of 5mM KH₂PO₄ and 1 mM triethylamine) adjusted to pH 5 with 1 N phosphoric acid, 200 ml acetonitrile, 50 ml methanol and 50 ml tetrahydrofuran.

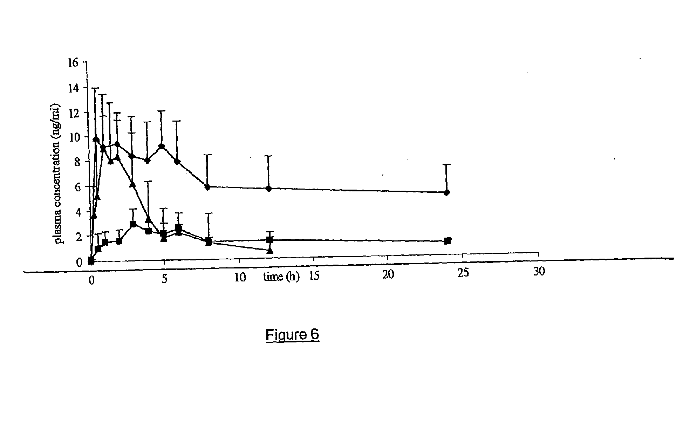

Figure 6 shows the average plasma propanolol concentration, as a function of time, after oral administration of 80 mg propranolol for each the matrix-in-pipe system of the invention (F-1) (◆), the core-in-capsule formulation (F-2) (▲) and Inderal^{®} retard mitis commercial formulation (■).

The mean area under the curve over 24 hours (AUC₀₋₂₄ₕ) - being a measure of bio-availability - of the matrix-in-pipe system of the invention was 4 times higher than for Inderal^{®} (35.8 ng ml⁻¹ h⁻¹).

## Claims

1. A biologically active composite solid shaped article comprising an outer layer and an inner core, wherein:
(a) the outer layer is a coating retaining the structural integrity of the inner core and consisting of :
- at least one polymeric component selected from the group consisting of hydrophobic cellulose polymers, acrylate (co)polymers, polyvinylpyrrolidone, polyethylene oxide, polyvinyl alcohol, poly(ethylene-co-vinyl acetate), and
- optionally at least one plasticizer for the said polymeric component,
(b) the inner core fills said outer layer and comprises:
- at least a biologically active ingredient, and
- an excipient for the said biologically active ingredient, said excipient comprising at least one hydrophilic cellulose polymer and an amphiphilic material, wherein the weight ratio of said hydrophilic cellulose polymer to said amphiphilic material is from 0.2 : 1 to 0.6 : 1.

2. A biologically active composite solid shaped article according to claim 1, wherein the hydrophilic cellulose polymer of the inner core is hydroxypropylmethylcellulose.

3. A biologically active composite solid shaped article according to claim 1 or claim 2, wherein the amphiphilic material of the inner core has both a portion derived from a glyceride and a portion derived from a polyethylene glycol ester.

4. A biologically active composite solid shaped article according to any of claims 1 to 3, wherein the weight ratio of the hydrophilic cellulose polymer to the amphiphilic material in the blend of the inner core is from 0.3 : 1 to 0.6 : 1.

5. A biologically active composite solid shaped article according to any of claims 1 to 4, wherein the content of the biologically active ingredient in the inner core is in a range from 0.1 to 50% by weight.

6. A biologically active composite solid shaped article according to any of claims 1 to 5, wherein the content of the hydrophilic cellulose polymer in the inner core is in a range from 10 to 40% by weight.

7. A biologically active composite solid shaped article according to any of claims 1 to 6, wherein the content of the amphiphilic material in the inner core is in a range from 30 to 85% by weight.

8. A biologically active composite solid shaped article according to any of claims 1 to 7, wherein the polymeric component of the outer layer is selected from the group consisting of hydrophobic cellulose polymers, acrylate (co)polymers, polyvinylpyrrolidone, polyethylene oxide, polyvinyl alcohol, poly(ethylene-co-vinyl acetate) and mixtures thereof.

9. A biologically active composite solid shaped article according to any of claims 1 to 8, wherein the plasticizer for the polymeric component of the outer layer is selected from the group consisting of glycerol, polyols, esters formed between glycerol and acetic acid, sugars, glycol glycoside, poly(ethylene glycol), fatty acids and esters thereof with polyethylene glycol, propylene glycol, butylene glycol, phtalate esters, sebacate esters, and mixtures thereof.

10. Use of a hydrophilic cellulose polymer in combination with an amphiphilic material, wherein the weight ratio of the said hydrophilic cellulose polymer to the amphiphilic material in the said combination is from 0.2:1 to 0.6:1, for manufacturing at least a portion of a biologically active article according to any of claims 1 to 9.

11. A process for making the core material of a biologically active article according to any of claims 1 to 9, comprising extruding a blend of at least a biologically active ingredient, at least a hydrophilic cellulose polymer and at least an amphiphilic material, the weight ratio of the hydrophilic cellulose polymer to the amphiphilic material in the said blend being from 0.2:1 to 0.6:1, at a temperature within the range from 20°C to 60°C.

12. A process for making of a biologically active formulation comprising an inner core and an outer layer, the said process comprising:
- extruding a blend of at least a biologically active ingredient, at least a hydrophilic cellulose polymer and at least an amphiphilic material, the weight ratio of the hydrophilic cellulose polymer to the amphiphilic material in the said blend being from 0.2:1 to 0.6:1, at a temperature within the range from 20°C to 60°C, and
- co-extruding the said blend with the components of the outer layer.

## Patentansprüche

1. Biologisch aktiver geformter Feststoffverbundgegenstand mit einer Außenschicht und einem Innenkern, wobei:
(a) die Außenschicht eine Beschichtung ist, die die strukturelle Integrität des inneren Kerns bewahrt und sie besteht aus:
- zumindest einem Polymerbestandteil, ausgewählt aus der Gruppe, bestehend aus hydrophoben Zellulosepolymeren, Acrylat (Co)polymeren, Polyvinylpyrrolidon, Polyethylenoxid, Polyvinylalkohol, Poly(ethylen-co-vinylacetat), und
- wahlweise zumindest einem Plastifizierungsmittel für den Polymerbestandteil,
(b) der innere Kern die äußere Schicht füllt und er umfasst:
- zumindest einen biologischen Wirkstoff, und
- einen Hilfsstoff für den biologischen Wirkstoff, wobei der Hilfsstoff zumindest ein hydrophiles Zellulosepolymer und ein amphiphiles Material umfasst, wobei das Gewichtsverhältnis des hydrophilen Zellulosepolymers zum amphiphilen Material 0,2:1 bis 0,6:1 beträgt.

2. Biologisch aktiver geformter Feststoffverbundgegenstand nach Anspruch 1,
wobei das hydrophile Zellulosepolymer des Innenkerns Hydroxypropylmethylzellulose ist.

3. Biologisch aktiver geformter Feststoffverbundgegenstand nach Anspruch 1 oder Anspruch 2,
wobei das amphiphile Material des Innenkerns sowohl einen von einem Glyzerid stammenden als auch von einem Polyethylenglykolester stammenden Anteil besitzt.

4. Biologisch aktiver geformter Feststoffverbundgegenstand nach einem der Ansprüche 1 bis 3,
wobei das Gewichtsverhältnis des hydrophilen Zellulosepolymers zum amphiphilen Material in der Mischung des Innenkerns 0,3:1 bis 0,6:1 beträgt.

5. Biologisch aktiver geformter Feststoffverbundgegenstand nach einem der Ansprüche 1 bis 4,
wobei der Gehalt des biologischen Wirkstoffs im Innenkern 0,1 bis 50 Gew.-% beträgt.

6. Biologisch aktiver geformter Feststoffverbundgegenstand nach einem der Ansprüche 1 bis 5,
wobei der Gehalt des hydrophilen Zellulosepolymers im Innenkern 10 bis 40 Gew.-% beträgt.

7. Biologisch aktiver geformter Feststoffverbundgegenstand nach einem der Ansprüche 1 bis 6,
wobei der Gehalt des amphiphilen Materials im Innenkern 30 bis 85 Gew.-% beträgt.

8. Biologisch aktiver geformter Feststoffverbundgegenstand nach einem der Ansprüche 1 bis 7,
wobei der Polymeranteil der Außenschicht ausgewählt ist aus der Gruppe, bestehend aus hydrophoben Zellulosepolymeren, Acrylat (Co)polymeren, Polyvinylpyrrolidon, Polyethylenoxid, Polyvinylalkohol, Poly(ethylen-cowinlyacetat) und Mischungen hiervon.

9. Biologisch aktiver geformter Feststoffverbundgegenstand nach einem der Ansprüche 1 bis 8,
wobei das Plastifizierungsmittel für den Polymerbestandteil der Außenschicht ausgewählt ist aus der Gruppe, bestehend aus Glyzerin, Polyolen, Estern zwischen Glyzerin und Essigsäure, Zuckern, Glykolglykosid, Poly(ethylenglykol), Fettsäuren und Estern hiervon mit Polyethylenglykol, Propylenglykol, Butylenglykol, Phthalatestern, Sebacatestern und Mischungen hiervon.

10. Verwendung eines hydrophilen Zellulosepolymers in Kombination mit einem amphiphilen Material, wobei das Gewichtsverhältnis des hydrophilen Zellulosepolymers zum amphiphilen Material in der Kombination 0,2:1 bis 0,6:1 beträgt, zur Herstellung zumindest eines Teils eines biologisch aktiven Gegenstands nach einem der Ansprüche 1 bis 9.

11. Verfahren zur Herstellung des Kernmaterials eines biologisch aktiven Gegenstands nach einem der Ansprüche 1 bis 9, umfassend das Extrudieren einer Mischung aus zumindest einem biologischen Wirkstoff, zumindest einem hydrophilen Zellulosepolymer und zumindest einem amphiphilen Material, wobei das Gewichtsverhältnis des hydrophilen Zellulosepolymeren zum amphiphilen Material in der Mischung 0,2:1 bis 0,6:1 bei einer Temperatur von 20°C bis 60°C beträgt.

12. Verfahren zur Herstellung einer biologisch aktiven Formulierung, umfassend einen Innenkern und eine Außenschicht, wobei das Verfahren umfasst:
- Extrudieren einer Mischung aus zumindest einem biologischen Wirkstoff, zumindest einem hydrophilen Zellulosepolymeren und zumindest einem amphiphilen Material,
wobei das Gewichtsverhältnis des hydrophilen Zellulosepolymeren zum amphiphilen Material in der Mischung 0,2:1 bis 0,6:1 bei einer Temperatur von 20°C bis 60°C beträgt, und
- Coextrudieren der Mischung mit den Bestandteilen der Außenschicht.

## Revendications

1. Objet de forme solide d'un composite biologiquement actif comprenant une couche externe et un noyau interne, dans lequel :
(a) la couche externe est un revêtement conservant l'intégrité structurale du noyau interne et constituée de :
- au moins un composé polymérique choisi dans le groupe constitué des polymères de cellulose hydrophobe, des (co)polymères d'acrylate, de la polyvinylpyrrolidone, de l'oxyde de polyéthylène, du polyalcool de vinyle, du poly(acétate d'éthylène-co-vinyle), et
- facultativement au moins un plastifiant pour ledit composé polymérique,
(b) le noyau interne remplit ladite couche externe et comprend :
- au moins un ingrédient biologiquement actif, et
- un excipient pour ledit ingrédient biologiquement actif, ledit excipient comprenant au moins un polymère de cellulose hydrophile et un matériau amphiphile, dans lequel le rapport de poids dudit polymère de cellulose hydrophile par rapport audit matériau amphiphile va de 0,2 : 1 à 0,6 : 1.

2. Objet de forme solide d'un composite biologiquement actif selon la revendication 1, dans lequel le polymère de cellulose hydrophile du noyau interne est l'hydroxypropylméthylcellulose.

3. Objet de forme solide d'un composite biologiquement actif selon la revendication 1 ou la revendication 2, dans lequel le matériau amphiphile du noyau interne possède à la fois une partie dérivée d'un glycéride et une partie dérivée d'un ester de polyéthylène glycol.

4. Objet de forme solide d'un composite biologiquement actif selon l'une quelconque des revendications 1 à 3, dans lequel le rapport de poids du polymère de cellulose hydrophile par rapport au matériau amphiphile dans le mélange du noyau interne va de 0,3 : 1 à 0,6 : 1.

5. Objet de forme solide d'un composite biologiquement actif selon l'une quelconque des revendications 1 à 4, dans lequel la teneur en ingrédient biologiquement actif dans le noyau interne se situe dans un domaine allant de 0,5 à 50 % par poids.

6. Objet de forme solide d'un composite biologiquement actif selon l'une quelconque des revendications 1 à 5, dans lequel la teneur en polymère de cellulose hydrophile dans le noyau interne se situe dans un domaine allant de 10 à 40 % par poids.

7. Objet de forme solide d'un composite biologiquement actif selon l'une quelconque des revendications 1 à 6, dans lequel la teneur en matériau amphiphile dans le noyau interne se situe dans un domaine allant de 30 à 85 % par poids.

8. Objet de forme solide d'un composite biologiquement actif selon l'une quelconque des revendications 1 à 7, dans lequel le composé polymérique de la couche externe est choisi dans le groupe constitué des polymères de cellulose hydrophobe, des (co)polymères d'acrylate, de la polyvinylpyrrolidone, de l'oxyde de polyéthylène, du polyalcool de vinyle, du poly(acétate d'éthylène-co-vinyle) et des mélanges de ceux-ci.

9. Objet de forme solide d'un composite biologiquement actif selon l'une quelconque des revendications 1 à 8, dans lequel le plastifiant pour le composé polymérique de la couche externe est choisi dans le groupe constitué du glycérol, des polyols, des esters formés entre le glycérol et l'acide acétique, des sucres, du glycol glycoside, du poly(éthylène glycol), des acides gras et des esters de ceux-ci avec du polyéthylène glycol, du propylène glycol, du butylène glycol, des esters de phtalate, des esters de sébacate et des mélanges de ceux-ci.

10. Utilisation d'un polymère de cellulose hydrophile en combinaison avec un matériau amphiphile, dans lequel le rapport de poids dudit polymère de cellulose hydrophile par rapport au matériau amphiphile dans ladite combinaison va de 0,2 : 1 à 0,6 : 1 pour la fabrication d'au moins une partie d'un objet biologiquement actif selon l'une quelconque des revendications 1 à 9.

11. Procédé pour la fabrication d'un matériau de noyau d'un objet biologiquement actif selon l'une quelconque des revendications 1 à 9, comprenant l'extrusion d'un mélange d'au moins un ingrédient biologiquement actif, d'au moins un polymère de cellulose hydrophile et d'au moins un matériau amphiphile, le rapport de poids du polymère de cellulose hydrophile par rapport au matériau amphiphile dans ledit mélange étant de 0,2 : 1 à 0,6 : 1, à une température comprise dans le domaine allant de 20 °C à 60 °C.

12. Procédé pour la fabrication d'une formulation biologiquement active comprenant un noyau interne et une couche externe, ledit procédé comprenant :
- l'extrusion d'un mélange d'au moins un ingrédient biologiquement actif, d'au moins un polymère de cellulose hydrophile et d'au moins un matériau amphiphile, le rapport de poids du polymère de cellulose hydrophile par rapport au matériau amphiphile dans ledit mélange allant de 0,2 : 1 à 0,6 : 1 à une température comprise dans le domaine allant de 20 °C à 60 °C, et
- la co-extrusion dudit mélange avec les composés de la couche externe.
